# EUROPÄISCHE PATENTSCHRIFT

(11) **EP 0 401 728 B1**
(45) Veröffentlichungstag und Bekanntmachung des Hinweises auf die Patenterteilung: **09.03.1994**
(21) Anmeldenummer: 90110564.3
(22) Anmeldetag: 05.06.1990
(51) Int. Cl.: A61B 17/32

(54) **Retrograd schneidende Hakenstanze**
Backwards cutting hook punch
Poinçon en forme de crochet à coupe rétrograde

(30) Priorität: 08.06.1989 DE 3918720
(43) Veröffentlichungstag der Anmeldung: 12.12.1990
(73) Patentinhaber: Richard Wolf GmbH, 75438 Knittlingen (DE)
(72) Erfinder: Hiltebrandt, Siegfried, D-7134 Knittlingen (DE); Dingler, Andreas, D-7534 Birkenfeld (DE); Falk, Ernst, D-7137 Sternenfels-Diefenbach (DE); Fischmeister, Martin Franz, Dr. Med, A-4040 Linz (AT)
(74) Vertreter: Wilcken, Thomas, Dipl.-Ing.

(56) Entgegenhaltungen:
- EP-A- 0 313 820
- DE-A- 3 303 349
- DE-U- 8 712 271
- DE-U- 8 810 968
- FR-B- 1 037 403

## Beschreibung

Die Erfindung geht von einer retrograd schneidenden Hakenstanze aus, bei der das eine mittels einer Zug- und Druckstange betätigte Maulteil retrograd im distalen Ende des mit dem Schaft starr verbundenen Maulteiles schwenkbeweglich gelagert ist, wie aus der DE-GM 8 712 271 und der DE-OS 35 23 022 bekannt ist.

Diese bekannten retrograd arbeitenden Instrumente dienen zum Entnehmen von Gewebeteilen oder auch zum Entfernen von Fremdkörpern sind aber nur beschränkt für die Arthroskopie geeignet, da im Kniegelenk für das Einführen der Scheren durch eine Trokarhülse nur ein sehr kleiner Raum zur Verfügung steht und daher nur eine stark begrenzte Manipulation ermöglicht, so daß eine Vielzahl von unterschiedlichen Instrumenten für Eingriffe im Kniegelenk erforderlich sind und im allgemeinen auch mehrere Einstiche durchgeführt werden müssen.

Die Aufgabe der Erfindung besteht darin, mit einer einzigen retrograd schneidenden Hakenstanze den wesentlichen Teil eines Eingriffes in einem Kniegelenk und vor allem den kritischen Bereich der Gelenkstelle insgesamt bearbeiten bzw. operieren zu können und dadurch mehrere Einstiche in das Kniegelenk ausschließen zu können.

Diese Aufgabe wird durch die Merkmale des Anspruches 1 gelöst.

Durch diese Lösung ist es möglich, die Hakenstanze durch eine Inzision in das Kniegelenk einzuführen, wobei man in der Lage ist, Teile des Gelenkes mit einem einzigen Instrument insgesamt operieren zu können, ohne daß besondere Manipulationen des Instrumentes erforderlich sind. Mit der erfindungsgemäßen Hakenstanze besteht die Möglichkeit eine Meniskusresektion bzw. den kritischen Bereich im Kniegelenk praktisch ohne wesentliche Positionsänderung bearbeiten zu können, wobei die visuelle Kontrolle mittels einer über einen Einstich in den aufgedehnten Gelenkraum eingeführten Optik erfolgt. Dabei ist das Instrument, um Verletzungen im Gelenkraum zu vermeiden, an seinen distalem Ende und im Bereich der retrograd gerichteten Maulteile atraumatisch ausgebildet. Durch die Ausbildung der Hakenstanze nach der Erfindung besitzt sie eine den anatomischen Gegebenheiten angepaßte Schneidenform, so daß nur äußerst geringe Positionsänderungen erforderlich sind. Weiter wird eine hohe Stabilität des Zangenmaules erreicht.

Die Erfindung wird nachstehend anhand der Zeichnung erläutert. Es zeigt:
- Figur 1: die Hakenstanze nach der Erfindung in Seitenansicht mit vergrößertem distalen Instrumentenende,
- Figur 2: eine vergrößerte Draufsicht auf das distale Ende der Ausführung nach Figur 1 jedoch mit geschlossenem Schneidenmaul,
- Figur 3: einen Schnitt nach der gebrochenen Linie III-III der Figur 2,
- Figur 4: einen Schnitt entlang der Längsachse nach Linie III-IV der Figur 2.

Die Hakenstanze besteht aus einem hohlen Schaft 1, der distal mit einem starren Maulteil 2 versehen ist und durch den eine Zug- und Druckstange 3 für das zweite verschwenkbare Maulteil 4 verläuft, die durch den Griffteil 5 betätigt wird, während der zweite Griffteil 6 starr mit dem proximalen Ende des Schaftes verbunden ist.

Das starre Maulteil 2 besteht aus einem geraden in Längsrichtung der Hakenstanze verlaufenden Teil 2a, der in einen zur Längsachse seitlich abgewinkelten Teil 2b übergeht. Der gerade Teil 2a ist mit einem Längsschlitz 7 versehen, von dem ein abgewinkelter Schlitzteil 7a abzweigt, dessen obere Begrenzungswand die Schneidkante 7b des starren Maulteiles bildet, mit der die Schneidkante des verschwenkbaren Maulteiles 4 zusammenarbeitet.

In dem axialen geraden Schlitzteil 7 ist der axial verlaufende Teil 4a des Maulteiles 4 um die Achse 8 verschwenkbar gelagert, und an diesen axial verlaufenden Teil 2a schließt sich der ebenfalls seitlich abgewinkelte Maulteil 4b etwa parallel zum starren abgewinkelten Maulteil 2c an. Der axial verlaufende Maulteil 4a ist mit einem nur nach unten offenen Längsschlitz 9 versehen, durch den das distal abgeflachte Ende der Zug- und Druckstange 3 verläuft, die bei 10 mit dem verschwenkbaren Maulteil 4 gelenkig verbunden ist. Die Außenflächen 4a des geraden verschwenkbaren Teiles sind in dem geraden Teil des Längsschlitzes 7 des starren Maulteiles unmittelbar geführt, so daß dadurch die Stabilität des Maules erhöht wird, durch die ein Auslenken des schwenkbaren Maulteiles 4 beim Durchschneiden von Geweben, Knorpeln oder zähen Teilen unterbunden wird. Die freien Enden 2c und 4c der beiden Maulteile 2 und 4 sind atraumatisch ausgebildet, um beim Einführen in das aufgeweitete Kniegelenk Verletzungen zu vermeiden.

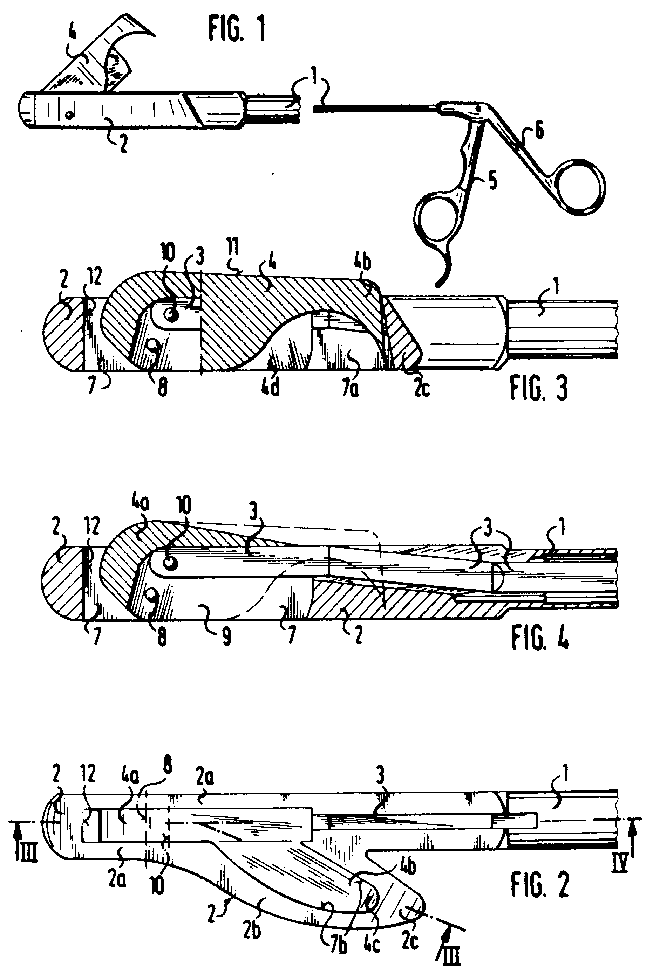

Die Hakenstanze wird durch eine Inzision in den aufgeweiteten Kniegelenkraum in geschlossenem Zustand eingeführt, wobei sich die Bodenfläche des nach unten offenen Schlitzes 9 des schwenkbaren Maulteiles 4 auf die als Anschlag dienende Oberkante des abgeflachten Teiles der Zug- und Druckstange 3 legt und verhindert, daß das Maulteil 4 nach unten aus dem Schlitzteil 7a des starren Maulteiles 2 herausragt. Zur Durchführung eines Eingriffes wird das Zangenmaul durch Betätigung mittels des Griffteiles 5 und der Zugstange 3 zur Verschwenkung des Maulteiles 4 entsprechend Figur 1 geöffnet, wobei die Öffnungsweite dadurch begrenzt wird, daß die Oberkante 11 des Maulteiles 4 sich gegen die Endfläche 12 des Langschlitzes 7 legt, wenn die Öffnungsweite erreicht ist.

## Patentansprüche

1. Retrograd schneidende Hakenstanze, bei der das eine mittels einer Zug- und Druckstange (3) betätigte Maulteil (4) retrograd im distalen Ende eines mit einem Schaft (1) starr verbundenen Maulteiles (2) schwenkbeweglich gelagert ist, dadurch gekennzeichnet, daß zur Durchführung von Eingriffen in einem Kniegelenk die beiden von der Drehachse (8) des schwenkbeweglich gelagerten Maulteiles (4) abgekehrten Enden (2c,4c) der Maulteile (2,4) seitlich im Winkel zur Längsachse der Hakenstanze angeordnet sind, wobei das zwischen zwei Anschlägen begrenzt verschwenkbare Maulteil (4) einen in einen Längsschlitz (7) des starren Maulteiles (2) verschwenkbaren axial gerichteten Teil (4a) aufweist, der mit einem zum Inneren der Hakenstanze offenen Längsschlitz (9) für die Aufnahme der Zug- und Druckstange (3) versehen ist.

2. Hakenstanze nach Anspruch 1, dadurch gekennzeichnet, daß die Oberkante der Zug- und Druckstange (3) im Bereich vor ihrer Anlenkung (10) an das bewegliche Maulteil (4) einen Anschlag für das bewegliche Maulteil in der Schließstellung und daß die distale abschließende Fläche (12) des Längsschlitzes (7) des starren Maulteiles (2) für die Oberfläche des beweglichen Maulteiles (4) einen Anschlag für die Öffnungsweite des beweglichen Maulteiles (4) bildet.

3. Hakenstanze nach Anspruch 1 oder 2, dadurch gekennzeichnet, daß das starre und das bewegliche Maulteil an ihrem freien Enden atraumatisch ausgebildet sind.

## Claims

1. A hook punch, cutting in a retrograde manner, in which the jaw part (4), which is actuated by means of a pull and push rod (3), is mounted so as to be pivotally movable in a retrograde manner in the distal end of a jaw part (2), rigidly connected with a shaft (1), characterised in that to carry out operations in a knee joint the two ends (2c,4c) of the jaw parts (2,4), facing away from the rotation axis (8) of the jaw part (4), mounted so as to be pivotally movable, are arranged laterally at an angle to the longitudinal axis of the hook punch, in which the jaw part (4), which is orientable to a limited extent between two stops, has an axially directed part (4a), orientable into a longitudinal slit (7) of the rigid jaw part (2), which part (4a) is provided with a longitudinal slot (9), open towards the interior of the hook punch, to receive the pull and push rod (3).

2. A hook punch according to Claim 1, characterised in that the upper edge of the pull and push rod (3) in the region in front of its articulation (10) to the movable jaw part (4) forms a stop for the movable jaw part in the closed position and that the distal terminating face (12) of the longitudinal slit (7) of the rigid jaw part (2) for the surface of the movable jaw part (4) forms a stop for the opening width of the movable jaw part (4).

3. A hook punch according to Claim 1 or 2, characterised in that the rigid and the movable jaw parts are constructed so as to be atraumatic at their free ends.

## Revendications

1. Poinçon en forme de crochet à coupe rétrograde dans lequel la partie de mors (4) commandée par une tige de traction et de compression (3) est montée à pivotement de manière rétrograde à l'extrémité distale de la partie de mors qui est raccordée de manière rigide avec un arbre (1), caractérisé en ce que, pour réaliser des interventions dans l'articulation de genou, les deux extrémités (2c, 4c) des parties de mors (2, 4) écartées de l'axe de rotation (8) de la partie de mors (4) montée de manière à pouvoir pivoter sont agencées latéralement en faisant un angle vis-à-vis de l'axe longitudinal du poinçon à crochet, la partie de mors (4) pivotant de manière limitée entre deux butées présentant une partie (4a) dirigée axialement et susceptible de pouvoir pivoter dans une fente longitudinale (7) de la partie de mors rigide (2), qui est pourvue d'une fente longitudinale (9) à l'intérieur du poinçon à crochet pour recevoir la tige de traction et de compression (3).

2. Poinçon à crochet selon la revendication 1, caractérisé en ce que la partie supérieure de la tige de traction et de compression (3) forme dans la zone située avant son articulation (10) sur la partie de mors mobile (4) une butée pour la partie de mors mobile en position de fermeture et la surface de fermeture distale (12) de la fente longitudinale (7) de la partie de mors rigide (2) forme pour la surface de la partie de mors mobile (4) une butée pour définir l'extension d'ouverture de la partie de mors mobile (4).

3. Poinçon à crochet selon la revendication 1 ou 2, caractérisé en ce que la partie de mors rigide et la partie de mors mobile sont conformée à leurs extrémités libres de manière atraumatique.
